# EUROPEAN PATENT APPLICATION

(11) **EP 3 683 300 A1**
(43) Date of publication of application: **22.07.2020**
(21) Application number: 20150746.4
(22) Date of filing: 08.01.2020
(51) Int. Cl.: C12M 3/10, C12M 1/00

(54) **SOFT SPHERE TRANSFER DEVICE AND RECOMBINANT PROTEIN PRODUCTION METHOD**

(30) Priority: 16.01.2019 JP 2019005019
(71) Applicant: Sinfonia Technology Co., Ltd., 1058564 Tokyo (JP)
(72) Inventor: TSUZUKI, Shoko, Tokyo, Tokyo 1058564 (JP)
(74) Representative: Angerhausen, Christoph

(57) **Abstract**

A soft sphere transfer device, includes: a water tank into which soft spheres are introduced; a transfer rotation member rotatably disposed in the water tank and including a plurality of accommodation grooves formed along a peripheral edge of the transfer rotation member; and an injection device configured to inject a predetermined substance into the soft spheres by piercing a needle into each of the soft spheres transferred to a needle piercing part by rotation of the transfer rotation member, wherein the water tank includes a pair of side walls facing each other in a width direction, and wherein a narrow width portion having a width equal to or smaller than a diameter of the soft spheres is provided on at least one portion in the width direction among portions of the side walls corresponding to the needle piercing part.

## Description

### TECHNICAL FIELD

The present disclosure relates to a soft sphere transfer device for transferring soft spheres such as fish eggs or the like in a water tank, and a recombinant protein production method.

### BACKGROUND

It is known that the use of eggs of a small fish such as a zebra fish or the like is beneficial in the technical field in which target substances such as recombinant proteins or the like are produced using genetic engineering technology by injecting genes into fertilized eggs. For example, when using a zebra fish to obtain a target substance, it is necessary to precisely inject a gene solution (vector) into a spherical fertilized egg having a diameter of 0.9 mm to 1.3 mm. There is known a microinjection technique in which, in order to make it difficult to damage such a fertilized egg, an extremely thin needle with a tip having a diameter of several to several tens µm is used to penetrate an egg membrane of a fertilized egg and to insert the tip of the needle into an embryo to inject a gene solution.

In this field, a microinjection work using a manipulator or the like that prevents hand shake in a manual work or a manual operation is a common method. However, it is difficult to process fertilized eggs in a quantity required for acquiring a practical amount of target substances. The injection operation is also limited in accuracy and stability. Therefore, various attempts have been made to achieve automation as described in the following three prior arts.

### [Prior Art Documents]

### Patent Documents

Patent Document 1: Japanese Patent No. 5647005
Patent Document 2: Japanese Patent No. 5823112
Patent Document 3: Japanese Patent No. 5787432

However, the aforementioned fertilized eggs frequently undergo cell division, for example, about every 30 minutes. In order to rapidly introduce a gene into an egg and obtain a target fertilized egg, it is necessary to process the fertilized egg with high efficiency. That is to say, the prior arts mentioned in the above patent documents are techniques based on the assumption of so-called batch processing in which the processing speed is restricted by the use of a predetermined container or the like. Therefore, the fertilized egg cannot always be performed in a state suitable for processing.

Thus, the inventor of the present disclosure has studied a fish egg transfer method that can perform predetermined processing to fish eggs with higher efficiency, and has found that, if a transfer rotation member having a plurality of accommodation grooves formed on the periphery thereof is provided in a water tank into which fish eggs are introduced and if the fish eggs are guided to the accommodation grooves of the transfer rotation member, it is possible to highly and efficiently perform predetermined processing on the fish eggs positioned in the accommodation grooves.

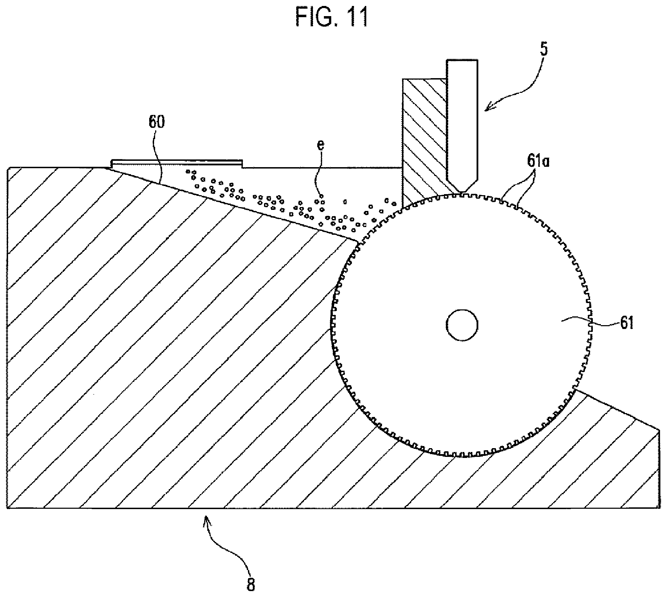

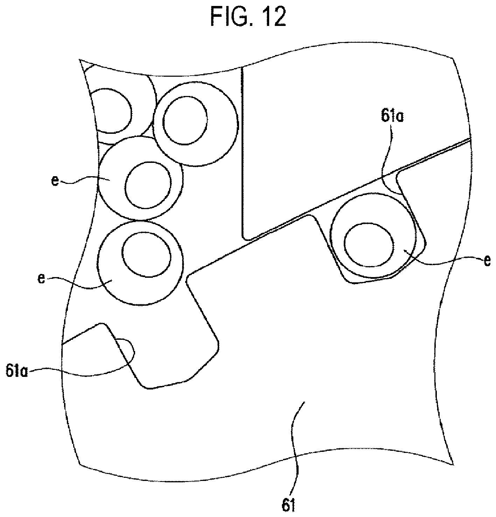

Specifically, as shown in FIGS. 11 and 12, an intermittently-rotating transfer rotation member 61 is disposed inside a water tank 8 filled with water up to the vicinity of its upper end, and a gene injection part 5 is disposed on the upper side of the water tank 8. In order to accommodate fish eggs e, the internal thickness of the water tank 8 is set such that a plurality of fish eggs e cannot be arranged in parallel. A plurality of accommodation grooves 61a for accommodating the fish eggs e one by one is provided at the peripheral edge portion of the transfer rotation member 61. Therefore, the fish eggs e introduced into the water tank 8 are guided to the peripheral edge portion of the transfer rotation member 61 by a guide 60, and the fish eggs e are supplied to the accommodation grooves 61a of the transfer rotation member 61 one by one. The gene injection part 5 injects a gene solution by inserting a needle into each of the fish eggs e separated and transferred one by one by the accommodation grooves 61a of the transfer rotation member 61.

In order to allow the fish eggs e to easily enter into an accommodation portion formed by the inner surfaces of the both side walls of the water tank 8 configured as above and the accommodation groove 61a, the size of the accommodation portion is set to be far larger than the diameter of the fish eggs e. Therefore, when the fish eggs e stored in the accommodation portion are being transferred, the fish eggs e are unexpectedly moved inside the accommodation portion. As a result, when the gene solution is injected into the transferred fish eggs e, the fish eggs e are not located at predetermined positions in the accommodation portion. Thus, the needle cannot be reliably pierced into the center (specifically, the embryo) of each of the fish eggs e. There may be a case where the gene solution cannot be injected into the fish eggs e.

### SUMMARY

The present disclosure provides a soft sphere transfer device and a recombinant protein production method capable of stably piercing a needle into a soft sphere such as a fish egg or the like.

A soft sphere transfer device according to the present disclosure, includes: a water tank into which soft spheres are introduced; a transfer rotation member rotatably disposed in the water tank and including a plurality of accommodation grooves formed along a peripheral edge of the transfer rotation member, the accommodation grooves extending in a width direction of the transfer rotation member to accommodate the soft spheres; and an injection device configured to inject a predetermined substance into the soft spheres by piercing a needle into each of the soft spheres transferred to a needle piercing part by rotation of the transfer rotation member, wherein the water tank includes a pair of side walls facing each other in the width direction such that the soft spheres are accommodated in the accommodation grooves of the transfer rotation member one by one, and wherein a narrow width portion having a width equal to or smaller than a diameter of the soft spheres is provided on at least one portion in the width direction among portions of the side walls corresponding to the needle piercing part.

According to the present disclosure, by providing the narrow width portion having a width equal to or smaller than the diameter of the soft spheres on at least one side in the width direction among the portions of both side walls corresponding to the gene injection part, it is possible to make sure that each of the soft spheres in the accommodation groove does not to move in the width direction at the narrow width portion. Therefore, it is possible to stably pierce the needle into each of the soft spheres.

Furthermore, in the soft sphere transfer device according to the present disclosure, the transfer rotation member may be configured to be intermittently driven and rotated at a predetermined pitch in a circumferential direction, and the transfer rotation member may be intermittently driven such that a stop position of each of the accommodation grooves of the transfer rotation member at the needle piercing part is more frontward in a rotational direction than the needle.

The soft sphere accommodated in the accommodation groove and moved by the rotation of the transfer rotation member is transferred in a state in which the soft sphere is pressed against a rear end of the accommodation groove in a rotation direction. Therefore, the center of the soft sphere accommodated in the accommodation groove is located rearward in the rotational direction. Thus, by intermittently driving the transfer rotation member such that the stop position of the accommodation groove of the transfer rotation member at the needle piercing part is more frontward in the rotational direction than the needle, it is easy for the needle to pierce the center of the soft sphere accommodated in the accommodation groove.

Furthermore, in the soft sphere transfer device according to the present disclosure, the transfer rotation member may be configured to be intermittently driven and rotated at a predetermined pitch in a circumferential direction, and the needle of the needle piercing part may be disposed such that a position of the needle is more rearward in a rotational direction than each of the accommodation grooves, which is stopped in the needle piercing part, of the transfer rotation member.

The soft sphere accommodated in the accommodation groove and moved by the rotation of the transfer rotation member is transferred in a state in which the soft sphere is pressed against the rear end of the accommodation groove of the rotation direction. Therefore, the center of the soft sphere accommodated in the accommodation groove is located rearward in the rotational direction. Thus, by disposing the needle so that a position of the needle of the needle piercing part is more rearward in a rotational direction than each of the accommodation grooves of the transfer rotation member stopped in the needle piercing part, it is easy for the needle to pierce the center of the soft sphere accommodated in the accommodation groove.

Furthermore, in the soft sphere transfer device according to the present disclosure, the narrow width portion may include a pair of protrusion portions protruding inward from both sides in the width direction of the side walls.

If the narrow width portion is composed of a pair of protrusion portions protruding inward from both sides in the width direction of the side walls as described above, the line through which the soft spheres are transferred can be made constant without being deflected in the width direction.

Furthermore, in the soft sphere transfer device according to the present disclosure, an inclined surface protruding inward toward a downstream side in a transfer direction of each of the protrusion portions may be provided at least on an upstream side in the transfer direction.

If an inclined surface protruding inward toward a downstream side in a transfer direction of each of the protrusion portions is provided at least on an upstream side in the transfer direction as described above, the soft sphere is smoothly guided to between the pair of protrusion portions without being caught at the upstream end of the protrusion portions in the transfer direction.

In addition, the present disclosure may be directed to a recombinant protein production method, including: recovering fish eggs, which are soft spheres transferred by the soft sphere transfer device, with a transfer container; transferring the fish eggs recovered into the transfer container in the step of recovering the fish eggs to a constant temperature bath and performing gene recombination for a predetermined time; sorting the fish eggs having proteins generated by the performing the gene recombination; and an extracting the proteins by grinding the fish eggs taken out in the step of sorting the fish eggs and putting the fish eggs into a centrifugal separator.

### BRIEF DESCRIPTION OF DRAWINGS

The accompanying drawings, which are incorporated in and constitute a part of the specification, illustrate embodiments of the present disclosure, and together with the general description given above and the detailed description of the embodiments given below, serve to explain the principles of the present disclosure.
FIG. 1 is a block diagram of a soft sphere processing apparatus including a soft sphere transfer device according to the present disclosure.
FIG. 2 is a side view showing the configuration of the soft sphere processing apparatus.
FIG. 3 is a side view of a device for collecting soft spheres from a breeding water tank that constitutes the soft sphere processing apparatus.
FIG. 4 is a side view of an unnecessary material separation part that constitutes the soft sphere processing apparatus.
FIG. 5 is a side view of a needle piercing part that constitutes the soft sphere processing apparatus.
FIG. 6 is a schematic plan view of the soft sphere processing apparatus as viewed from above.
FIG. 7 is an enlarged side view of essential portions of the needle piercing part that constitutes the soft sphere processing apparatus.
FIG. 8 is an enlarged side view of the surroundings of accommodation grooves of the needle piercing part that constitutes the soft sphere processing apparatus.
FIG. 9 is a plan view of the accommodation groove of the needle piercing part.
FIG. 10 is a sectional view taken along line X-X in FIG. 9.
FIG. 11 is a side view showing the surroundings of accommodation grooves of a conventional needle piercing part.
FIG. 12 is a side view showing conventional accommodation grooves.

### DETAILED DESCRIPTION

Reference will now be made in detail to various embodiments, examples of which are illustrated in the accompanying drawings. In the following detailed description, numerous specific details are set forth in order to provide a thorough understanding of the present disclosure. However, it will be apparent to one of ordinary skill in the art that the present disclosure may be practiced without these specific details. In other instances, well-known methods, procedures, systems, and components have not been described in detail so as not to unnecessarily obscure aspects of the various embodiments.

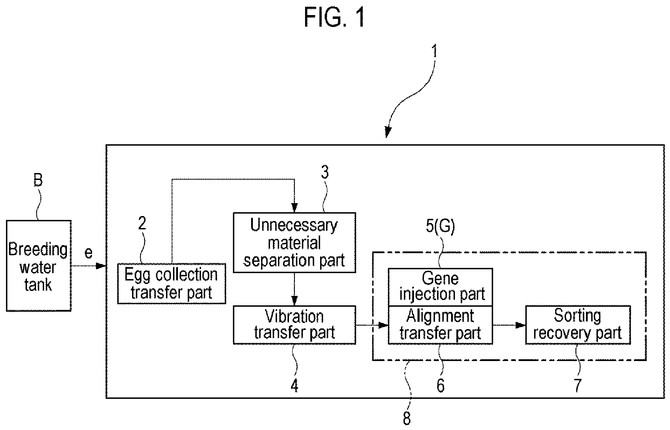

FIG. 1 shows a block diagram of a gene injection apparatus (soft sphere processing apparatus) 1 which is a fish egg processing apparatus provided with a soft sphere transfer device (in the present embodiment, a fish egg transfer device) according to the present disclosure. The gene injection apparatus 1 is an apparatus for promptly injecting a gene solution G containing a gene as a predetermined substance into a fertilized egg which is an egg e collected from a breeding water tank B, by piercing a needle into the fertilized egg. Then, in the fish egg e injected with the gene solution G, cell division is repeated subsequently and a protein derived from the base sequence of the injected gene is synthesized. The synthesized protein is collected, extracted and purified at a predetermined timing and is used, for example, for drug discovery research and mass production.

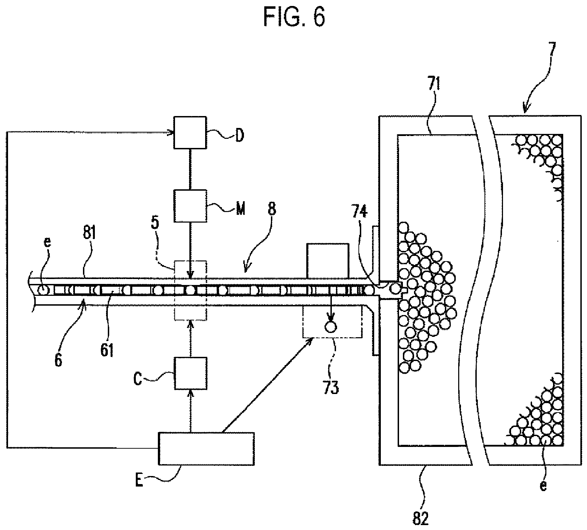

As shown in FIGS. 1 to 5, the gene injection apparatus 1 includes an egg collection transfer part 2 continuous to a breeding water tank B, an unnecessary material separation part 3 configured to separate the transferred fish eggs e from breeding water w and unnecessary materials such as excrement and residual food bg and sg, and the like, a vibration transfer part 4 configured to transfer the fish eggs e separated from the unnecessary materials bg and sg mainly by vibration, an alignment transfer part 6 configured to transfer the fish eggs e transferred by the vibration transfer part 4 while aligning the fish eggs e in a predetermined state, a gene injection part 5 as a needle piercing part configured to inject a gene solution G into the fish eggs e by piercing a needle into each of the fish eggs e transferred by the alignment transfer part 6, and a sorting recovery part 7 configured to efficiently collect the fish eggs e injected with the gene solution G. In the present embodiment, as shown in FIG. 6, the gene injection part 5, the alignment transfer part 6 and the sorting recovery part 7 are arranged in a processing water tank 8 having a substantially T-shape in a plan view.

In the present embodiment, a substantially spherical zebra fish egg having a diameter of about 1 mm is used as an example of the fish egg e. Since a fish is a vertebrate, it is easy to obtain a protein that can be used for drug discovery by gene introduction. In particular, a zebra fish is known as a species that can easily obtain fish eggs e as fertilized eggs from a breeding water tank B.

Hereinafter, the configuration of each part of the gene injection apparatus 1 will be described.

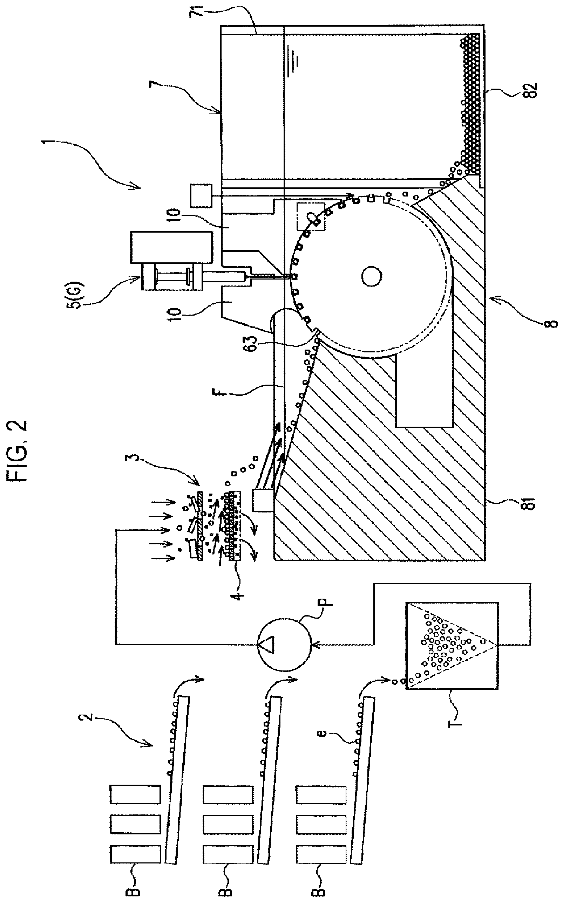

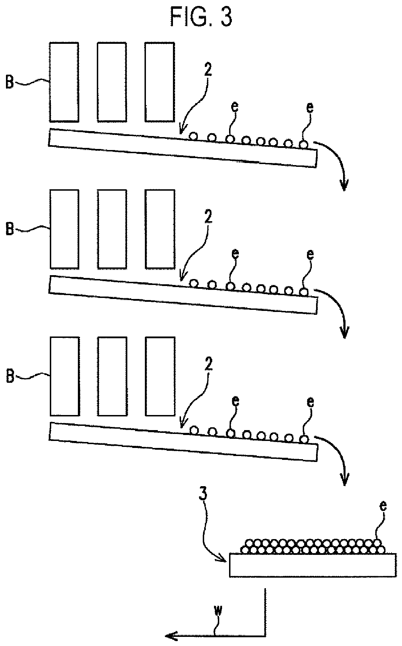

As shown in FIGS. 2 and 3, the egg collection transfer part 2 is, for example, a water conduit-shaped passage configured in a slope shape so as to efficiently collect fish eggs e from a plurality of breeding water tanks B arranged in parallel in a vertical direction. In the egg collection transfer part 2, the fish eggs e and the breeding water w which has been breeding fishes are simultaneously transferred from the breeding water tank B. The fish eggs e and the breeding water w are once stored in a tank T. Then, the fish eggs e and the breeding water w are raised by a pump P and are guided to the unnecessary material separation part 3. Specifically, the fish eggs e and the breeding water w are dropped from above and guided to the unnecessary material separation part 3 in a state where the falling energy is applied thereto. The tank T functions as a temporary cushioning tank for the collected fish eggs e and the breeding water w. In the illustrated example, for example, a spiral water flow is generated inside the tank T, whereby water can be continuously supplied from the pump P without delay. Furthermore, as a specific aspect, the breakage or wear of the pump P due to idle feeding is prevented by detecting a lower limit of a water level in the tank T, and the overflow is detected by detecting an upper limit of a water level. When the overflow actually occurs, the breeding water w is returned to the breeding water tank B via a pipe (not shown) leading to the breeding water tank B. The tank T and the pump P may be omitted if the breeding water tank B can be laid out at a sufficiently high position and if the fish eggs e can freely fall from the breeding water tank B to the unnecessary material separation part 3. In FIG. 3, the tank T and the pump P shown in FIG. 2 are omitted.

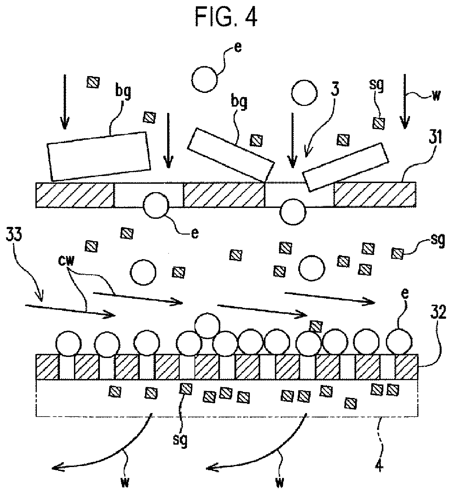

As specifically shown in FIG. 4, the unnecessary material separation part 3 includes a first net device 31 for collecting and removing unnecessary materials bg larger than the fish eggs e by passing the fish eggs e, a second net device 32 having a mesh which is supported by the vibration transfer part 4 and which prevents the fish eggs e from passing therethrough, and a purified water spray means 33 for spraying purified water cw to the fish eggs e present in the second net device 32. In the present embodiment, there is adopted a configuration in which the breeding water w crushed by passing through the second net device 32 or colliding with the second net device 32 is separated from, for example, the unnecessary materials sg smaller than the fish eggs e and passed through the second net device 32 and is introduced again to the breeding water tank B. Furthermore, in the present embodiment, the purified water spray means 33 is configured to more rapidly transfer the fish eggs e by spraying purified water cw to the fish eggs e along the direction in which the fish eggs e are transferred by the vibration transfer part 4. That is to say, in the present embodiment, there is adopted a configuration in which the breeding water w passed through the second net device 32 and possibly mixed with bacteria, microorganisms and the like is not introduced into the processing device disposed in the processing water tank 8. The purified water cw used in the present embodiment refers to not only distilled water or tap water but also water having less contamination than the breeding water w.

The vibration transfer part 4 is configured to guide the fish eggs e to the processing water tank 8 by providing a predetermined vibration to the second net device 32. The fish eggs e to which the vibration is applied by the vibration transfer part 4 are transferred to and put into the processing water tank 8 quickly and efficiently.

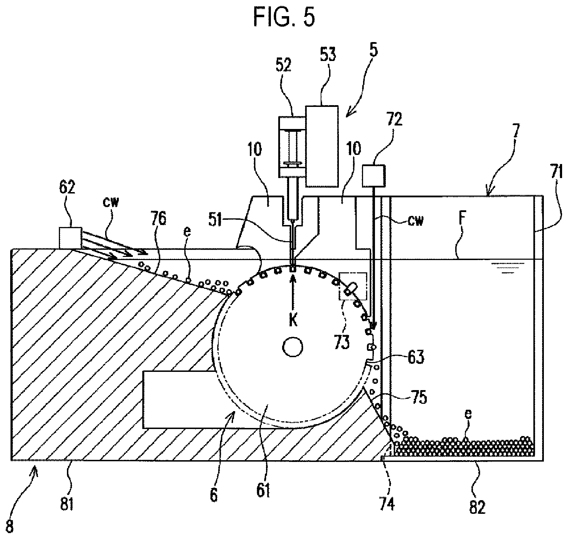

As shown in FIGS. 5 and 6, in the present embodiment, the gene injection part 5, the alignment transfer part 6 and the sorting recovery part 7 are provided in the processing water tank 8. Specifically, the gene injection part 5 is disposed above the processing water tank 8, and the alignment transfer part 6 and the sorting recovery part 7 are disposed inside the processing water tank 8. Moreover, as shown in FIGS. 2 and 5, water is filled up to the vicinity of the upper end of the processing water tank 8. In addition, symbol F indicated in FIGS. 2 and 5 denotes an upper surface of water.

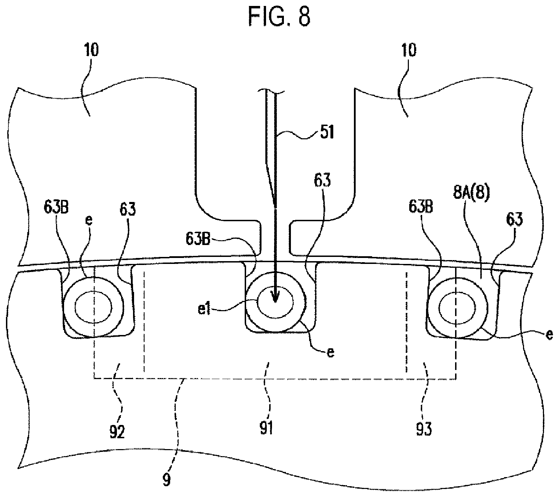

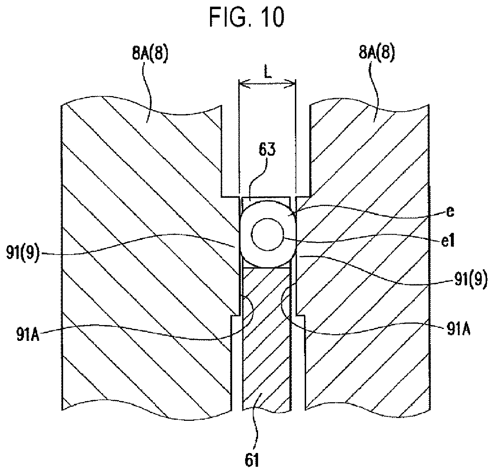

The processing water tank 8 is made of, for example, a synthetic resin material, a glass material or the like and is configured to process the collected fish eggs e.g., The gene injection part 5 for injecting a predetermined substance into the fish eggs e and the alignment transfer part 6 are provided in the processing water tank 8. Specifically, as shown in FIG. 6, the processing water tank 8 includes a pre-processing area 81 having a spacing between the inner surfaces of a pair of side walls 8A and 8A (*see* FIGS. 8 to 10) facing each other in the transfer width direction (hereinafter simply referred to as width direction) so as to accommodate the unprocessed fish eggs e one by one, and a post-processing area 82 for accommodating the fish eggs e subjected to predetermined processing. Specifically, the processing water tank 8 has a substantially T-shape in a plan view in which the pre-processing area 81 and the post-processing area 82 are continuous to each other with the spacing (distance) between the inner surfaces of both side walls 8A and 8A set such that a plurality of fish eggs e cannot be arranged in parallel in the width direction. In the present embodiment, in order to make visible at least an injection position (processing position) K at which the gene solution G is injected by piercing each of the fish eggs e with a needle, the portion of the processing water tank 8 corresponding to the injection position K is made of a transparent resin material, a glass material or the like. The transfer width direction refers to a direction orthogonal to the transfer direction. In FIG. 6, the transfer width direction is an up-down direction in a plan view.

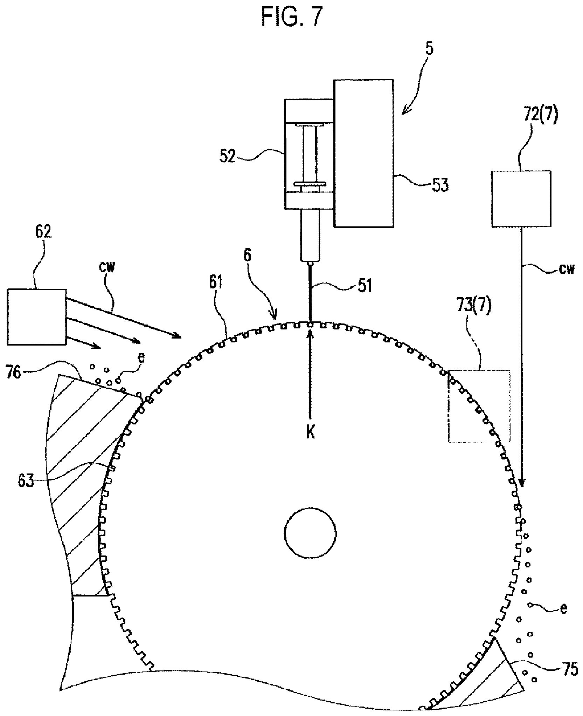

As shown in FIG. 7, the alignment transfer part 6 is configured to align the eggs e introduced into the pre-processing area 81 so as to be easily processed by the gene injection part 5. The alignment transfer part 6 includes a transfer rotation member 61 as a disc having a disc shape and provided with a plurality of accommodation grooves 63 formed at a predetermined pitch (an equal pitch in the present embodiment) on the periphery of the transfer rotation member 61, and an alignment pump 62 configured for spraying purified water cw toward the transfer rotation member 61. The fish eggs e introduced onto the guide 76 provided at upstream end of the pre-processing area 81 in the transfer direction are smoothly guided to the transfer rotation member 61 by the purified water cw supplied by the alignment pump 62 and are positioned in the accommodation grooves 63. The guide 76 has an inclined surface that becomes lower toward the downstream side in the transfer direction. In the present embodiment, as shown in FIG. 6, the transfer rotation member 61 is precisely controlled in rotation by driving an AC servomotor M via a motor driver D. The motor driver D is controlled by a control device E. As long as the fish eggs e can be arranged and transferred one by one, the transfer rotation member 61 may be in the shape of, for example, a belt or a chain capable of forming an endless track. Each of the accommodation grooves 63 is formed of a concave groove extending in the width direction of the transfer rotation member 61. The width direction movement of the fish egg e accommodated in each of the accommodation grooves 63 is restricted by both side walls 8A and 8A. In other words, an accommodation portion for accommodating each of the fish eggs e is defined by the inner surfaces of the side walls 8A and 8A and each of the accommodation grooves 63. In addition, the width direction dimension of the transfer rotation member 61 is set to a width dimension smaller than a gap between narrow width portions 9 and 9 so that the transfer rotation member 61 can pass between the narrow width portions 9 and 9 to be described below.

The transfer rotation member 61 is configured to be intermittently driven and rotated at a predetermined a predetermined pitch (an equal pitch in the present embodiment) (actually, the same pitch as the pitch between the accommodation grooves 63 adjacent to each other in the rotation direction) in the circumferential direction by the AC servomotor M controlled by the above-described control device E. Then, each time when the AC servomotor M is driven, the rotation of the transfer rotation member 61 is controlled such that the accommodation groove 63 is located at a predetermined position with respect to the gene injection part 5. Furthermore, the transfer rotation member 61 is intermittently driven by the AC servomotor M so that the stop position of the accommodation groove 63 of the transfer rotation member 61 in the gene injection part (needle piercing part) 5 is more frontward in the rotational direction than a capillary (needle) 51 to be described below (*see* FIG. 8). Although the driving of the AC servomotor M is controlled in the present embodiment, the capillary (needle) 51 may be disposed such that the position of the capillary (needle) 51 of the gene injection part (needle piercing part) 5 with respect to the accommodation groove 63 of the transfer rotation member 61 stopped in the gene injection part (needle piercing part) 5 is more rearward in the rotational direction than the capillary (needle) 51.

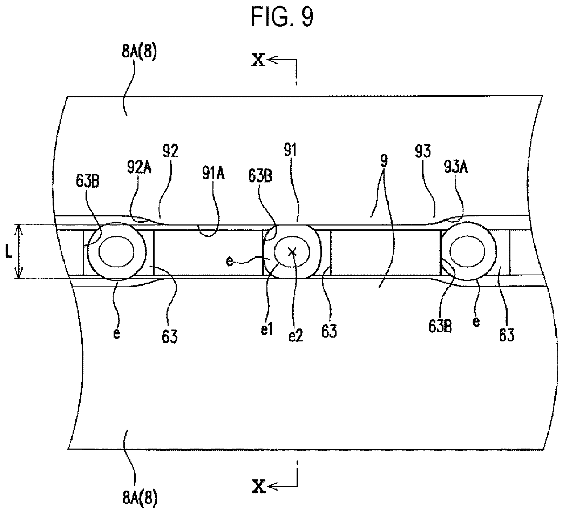

The fish egg e accommodated in the accommodation groove 63 and moved by the rotation of the transfer rotation member 61 as described above is transferred in a state in which the fish egg e is pressed against a rear end 63B of the accommodation groove 63 in the rotation direction (*see* FIGS. 8 and 9). Therefore, the center (specifically, the center of the embryo e1) of the fish egg e accommodated in the accommodation groove 63 is located rearward in the rotational direction. Thus, by intermittently driving the transfer rotation member 61 so that the stop position of the accommodation groove 63 of the transfer rotation member 61 in the gene injection part (needle piercing part) 5 is more frontward in the rotational direction than the capillary (needle) 51 as described above, it is easy for the capillary (needle) 51 to pierce the center of the fish egg e accommodated in the accommodation groove 63 and it is possible for the capillary (needle) 51 to reliably pierce the fish egg e. Even when the capillary (needle) 51 is disposed such that the position of the capillary (needle) 51 of the gene injection part (needle piercing part) 5 with respect to the accommodation groove 63 of the transfer rotation member 61 stopped in the gene injection part (needle piercing part) 5 is more rearward in the rotational direction than the capillary (needle) 51, it is possible to obtain the same effect as in the case of intermittently driving the transfer rotation member 61 so that the stop position of the accommodation groove 63 is more frontward in the rotational direction than the capillary (needle) 51.

The gene injection part 5 is an injection device for injecting the gene solution G into the fish egg e transferred by the alignment transfer part 6. The gene injection part 5 includes a substantially needle-shaped capillary 51 configured to directly inject a gene into the fish egg e, a syringe pump 52 for supplying a predetermined amount of gene to the capillary 51, and a positioner 53 for positioning the capillary 51 and the syringe pump 52 in the vertical direction (Z direction). The positioner 53 can also adjust the positions of the capillary 51 and the syringe pump 52 in the front-rear and left-right directions (X and Y directions). The capillary 51 corresponds to a tubular needle capable of introducing a predetermined substance into the fish egg e at a predetermined timing. In addition, a guide 10 for confining the eggs e in proximity to the accommodation groove 63 is provided in a predetermined region in the front or the rear of the injection position (processing position) K at which the gene solution G is injected into the egg e by the gene injection part 5 (*see* FIG. 8). That is to say, the fish egg e is introduced into the accommodation groove 63 at a position where the guide 10 does not exist, and is moved under the guide 10 along with the rotation of the transfer rotation member 61, whereby the removal of the fish egg e from the accommodation groove 63 is prevented. In addition, for example, after the capillary (needle) 51 is lowered at the injection position (processing position) K to pierce the fish egg e to inject the gene into the fish egg e, the fish egg e adhering to the capillary (needle) 51 and tending to be lifted at the time of raising the capillary (needle) 51 can be separated from the capillary (needle) 51 by the guide 10 and can be returned to the accommodation groove 63. In addition, after injecting the gene, the fish egg e reaches a position where the guide 10 does not exist, and comes into a state in which the fish egg e can be removed from the accommodation groove 63.

As shown in FIGS. 8 to 10, narrow width portions 9 and 9 having a width equal to or smaller than the diameter of the fish egg e are provided on both sides in the width direction among the portions of both side walls 8A and 8A of the pre-processing area 81 corresponding to the gene injection part (needle piercing part) 5. The narrow width portions (protrusion portions) 9 and 9 are composed of a pair of protrusion portions that protrude inward from both sides in the width direction of the side walls 8A and 8A. Each protrusion portion 9 includes a protruding main body portion 91 positioned at the center in the transfer direction and having a flat surface 91A, a first guide portion 92 provided on the upstream side of the protruding main body portion 91 in the transfer direction and having a first inclined surface 92A protruding inward toward the downstream side, and a second guide portion 93 provided on the downstream side of the protruding main body portion 91 in the transfer direction and having a second inclined surface 93A retreating outward toward the downstream side. In this regard, the distance between the narrow width portions 9 and 9 (the distance between the flat surfaces 91A and 91A) L is set to become to a minimum diameter of 0.9 mm with respect to the diameter of the fish egg e of 0.9 mm to 1.3 mm. By virtue of this setting, it is possible to cope with a fish egg e having a minimum diameter. The narrow width portions 9 and 9 may be integrally formed with the side walls 8A and 8A, or may be separately formed and attached to the side walls 8A and 8A.

By providing the narrow width portions 9 and 9 having a width equal to or smaller than the diameter of the fish egg e on both sides in the width direction of the portions of both side walls 8A and 8A corresponding to the gene injection part (needle piercing part) 5 as described above, it is possible to make sure that the fish egg e in the accommodation groove 63 does not to move in the width direction at the narrow width portions 9 and 9. This enables the capillary (needle) 51 to stably pierce the center e2 (*see* FIG. 9) of the embryo e1 of the fish egg e. In addition, the narrow width portions 9 and 9 are provided only in the gene injection part 5. Therefore, as compared with a case where the narrow width portions 9 and 9 are provided on the entire side walls 8A and 8A, it is possible to reduce the possibility that the fish egg e passing through the narrow width portions 9 and 9 is damaged. In particular, the present disclosure is effective for fish eggs e larger than the gap between the narrow width portions 9 and 9. Furthermore, the narrow width portions 9 and 9 are formed of a pair of protrusion portions protruding from both sides in the width direction of the side walls 8A and 8A. Therefore, the line along which the fish egg e is transferred can be made uniform without deflection in the width direction. Furthermore, the first inclined surface 92A protruding inward toward the downstream side is provided on the upstream side of each protrusion portion 9 in the transfer direction. Therefore, the width between the protrusion portions 9 and 9 is gradually reduced toward the downstream side. Thus, the fish egg e is not caught on the transfer direction upstream end of the protrusion portions 9 and 9 and is smoothly guided to between the protrusion portions 9 and 9. Furthermore, the second inclined surface 93A retreating outward toward the downstream side is provided on the transfer direction downstream side of each protrusion portion 9. Therefore, it is possible to gradually release the pressing force applied from the narrow width portions 9 and 9 to the fish egg e. In this regard, the first inclined surface 92A and the second inclined surface 93A may be formed in a gentle arc shape, a straight line shape or a smooth wave shape. The shapes of the first inclined surface 92A and the second inclined surface 93A may be any shape as long as the fish egg e is not caught.

Furthermore, the sorting recovery part 7 for efficiently recovering the fish eggs e injected with genes is provided from the alignment transfer part 6 to the post-processing area 82.

The sorting recovery part 7 corresponds to a recovery device or recovery tank that recovers the processed fish eggs e. As shown in FIGS. 5 and 6, the sorting recovery part 7 includes a recovery container 71 as a recovery tank accommodated in the post-processing area 82, a recovery pump 72 provided in the pre-processing area 81 near the post-processing area 82 and configured to inject purified water cw toward the transfer rotation member 61, a sorting part 73 for discharging the fish egg e, into which the gene could not be correctly introduced in the gene injection part 5 constituting the processing device, to the outside of the processing water tank 8 through an NG egg removal path, and a guide 75 provided so as to cover the lower half of the transfer rotation member 61 in the processing water tank 8 and configured to guide the fish egg e from the pre-processing area 81 to an OK egg recovery path leading to the post-processing area 82. The OK egg recovery path is formed along the guide 75. In the recovery container 71 according to the present embodiment, by providing a slit 74 in the portion which faces the post-processing area 82, the fish egg e moved from the pre-processing area 81 can be efficiently introduced into the recovery container 71 through the slit 74. Furthermore, in the present embodiment, by allowing a part of the guide 75 to enter the recovery container 71, the fish egg e can be efficiently guided to the recovery container 71.

The sorting part 73 includes a removal-purpose pushing water flow pump (not shown), a pushing water input/output pipe (not shown) as an NG egg removal path, and an electromagnetic valve (not shown) configured to open and close the pushing water input/output pipe. The electromagnetic valve is opened when the accommodation groove 63 containing the egg e determined to be NG is moved to the removal position. The fish egg e determined to be NG is pushed by the water flow and is removed from the accommodation groove 63. The fish egg e is sent to the pushing water input/output pipe and is sorted.

In the present embodiment, as shown in FIGS. 5, 6 and 7, there is adopted a configuration in which the fish eggs e introduced into the processing water tank 8 are processed with higher efficiency. That is to say, the fish eggs e introduced into the pre-processing area 81 of the processing water tank 8 are smoothly guided to the accommodation grooves 63 by the purified water cw sprayed from the alignment pump 62. Then, the fish eggs e accommodated in the accommodation grooves 63 are moved to the position directly below the capillary 51 by the rotation of the transfer rotation member 61 without the order being changed. In this regard, the width of the side walls 8A and 8A in the portion facing the pre-processing area 81 and the OK egg recovery path is set to a dimension including the processing portion K such that two or more fish eggs e cannot be arranged side by side. Furthermore, the portions of the side walls 8A and 8A facing pre-processing area 81 and the OK egg recovery path are formed of transparent members including the processing position K. That is to say, the fish eggs e guided to the position directly below the capillary 51 are necessarily kept in a state in which the fish eggs e can be clearly visually recognized one by one. Since the position of the embryo e1 (*see* FIG. 8) inside the fish egg e can be clearly visually recognized by a camera C illustrated in FIG. 6, the capillary 51 can accurately inject a gene into the embryo e1 of each of the fish eggs e while the camera C and the gene injection apparatus 1 are being precisely controlled. At this time, since the transfer rotation member 61 is formed in a substantially circular shape and the accommodation grooves 63 are arranged at equal pitches (equal intervals) in the circumferential direction, the accommodation groove 63 is accurately located directly below the capillary 51 by the control device E. In this regard, if the fish egg e is not suitable for gene injection, or if it is possible for the camera C to visually recognize that the gene could not be accurately injected into the fish egg e, it is possible to separately collect the fish egg e by allowing the fish egg e to pass through the sorting part 73 provided at the position where the transfer rotation member 61 is rotated by a predetermined angle. This prevents unnecessary leakage to the outside of the fish egg e that may have undergone recombination. In addition, the fish eggs e accurately injected with genes at a high percentage are guided to the downstream side of the post-processing area 82. Furthermore, the fish eggs e injected with genes by the capillary 51 are reliably separated from the accommodation grooves 63 by the purified water cw sprayed from the recovery pump 72. Then, these fish eggs e are smoothly guided from the post-processing area 82 to the OK egg recovery path and are accommodated in the recovery container 71 through the slit 74. When imaging the embryo e1 (*see* FIG. 8) with the camera C, the narrow width portions 9 and 9 prevent the fish egg e from moving in the width direction. This provides an effect that the fish egg e can be imaged more clearly by the camera C.

Furthermore, the present disclosure may be directed to a recombinant protein production method including: a recovery step of recovering fish eggs e, which are soft spheres transferred by the soft sphere transfer device, with a transfer container (not shown); a gene recombination step of transferring the fish eggs e recovered into the transfer container in the recovery step to a constant temperature bath (having a temperature of, e.g., 28 degrees C) and performing gene recombination for a predetermined time (e.g., about 24 hours); an expression egg sorting step of sorting fish eggs e having proteins generated in the gene recombination step; and an extraction step of extracting proteins by grinding the fish eggs e taken out in the expression egg sorting step and putting the fish eggs e into a centrifugal separator. Therefore, the fish eggs e recovered (accommodated) in the transfer container in the recovery step are put into the constant temperature bath at 28 degrees C for about 24 hours in the gene recombination step and are subjected to gene recombination. Then, the proteins can be extracted by taking out the fish eggs e having the proteins in the expression egg sorting step and by grinding the taken-out fish eggs e and putting the fish eggs e into the centrifugal separator in the extraction step. In addition, the sorting device used in the expression egg sorting step has a configuration obtained by removing the gene injection part 5 from the soft sphere processing apparatus 1 shown to FIGS. 5 and 6. The fish eggs e processed by the soft sphere processing apparatus 1, i.e., the fish eggs e recovered in the transfer container, are transferred to the constant temperature bath (having a temperature of, e.g., 28 degrees C). The fish eggs e subjected to gene recombination for a predetermined time (e.g., about 24 hours) are put into a pre-sorting area of an expression egg sorting device corresponding to the pre-processing area. By virtue of the configuration corresponding to the camera C, the expression eggs are sorted from a fish egg group including expression eggs, non-expression eggs and dead eggs. In some cases, the extracted proteins may be purified by chemical treatment. Furthermore, the extracted proteins may be processed with the charge of ions in order to finely separate the extracted proteins.

In addition, the soft sphere transfer device according to the present disclosure is not limited to the above embodiment, but may be variously modified without departing from the scope of the present disclosure. For example, the soft spheres of the present disclosure are not limited to the fish eggs e, but may be, for example, artificial salmon roes or soft spheres other than the fish eggs e.

In the above-described embodiment, the first guide portion 92 and the second guide portion 93 are provided in the narrow width portions 9 and 9. However, the second guide portion 93 existing on the rear side in the transfer direction may be omitted. In some cases, both the first guide portion 92 and the second guide portion 93 may be omitted.

Moreover, in the above-described embodiment, a pair of narrow width portions 9 and 9 is provided in the width direction. However, a narrow width portion 9 may be provided only on one side.

Furthermore, in the above-described embodiment, the distance between the narrow width portions 9 and 9 is set to become to a minimum diameter of 0.9 mm with respect to the diameter of the fish egg e of 0.9 mm to 1.3 mm. However, the distance between the narrow width portions 9 and 9 may be set to an average value of the diameters of all the fish eggs (for example, 1.1 mm or 84.6% of the maximum diameter of the fish eggs e).

Furthermore, in the above-described embodiment, the gene as a predetermined substance is injected into the fish egg. However, the soft sphere is not limited to the gene. Cells such as human cancer cells or the like, chemicals such as drugs, drug candidate substances, toxic substances or the like, food additives such as seasonings, colorants or the like, and other substances may be injected into the soft sphere.

According to the present disclosure, by providing the narrow width portion having a width equal to or smaller than the diameter of the soft sphere on at least one side in the width direction of the portions of both side walls corresponding to the needle piercing part, it is possible to make sure that the soft sphere in the accommodation groove does not move in the width direction at the narrow width portion. Therefore, it is possible to provide a soft sphere transfer device and a recombinant protein production method capable of stably piercing a soft sphere such as a fish egg or the like with a needle.

While certain embodiments have been described, these embodiments have been presented by way of example only, and are not intended to limit the scope of the disclosures. Indeed, the embodiments described herein may be embodied in a variety of other forms. Furthermore, various omissions, substitutions and changes in the form of the embodiments described herein may be made without departing from the scope of the disclosures. The accompanying claims and their equivalents are intended to cover such forms or modifications as would fall within the scope of the disclosures.

## Claims

1. A soft sphere transfer device, comprising:
a water tank (8) into which soft spheres are introduced;
a transfer rotation member (61) rotatably disposed in the water tank (8) and including a plurality of accommodation grooves (63) formed along a peripheral edge of the transfer rotation member (61), the accommodation grooves (63) extending in a width direction of the transfer rotation member (61) to accommodate the soft spheres; and
an injection device configured to inject a predetermined substance into the soft spheres by piercing a needle (51) into each of the soft spheres transferred to a needle piercing part (5) by rotation of the transfer rotation member (61),
wherein the water tank (8) includes a pair of side walls (8A) facing each other in the width direction such that the soft spheres are accommodated in the accommodation grooves (63) of the transfer rotation member (61) one by one, and
wherein a narrow width portion (9) having a width equal to or smaller than a diameter of the soft spheres is provided on at least one portion in the width direction among portions of the side walls (8A) that correspond to the needle piercing part (5).

2. The soft sphere transfer device of Claim 1, wherein the transfer rotation member (61) is configured to be intermittently driven and rotated at a predetermined pitch in a circumferential direction, and
wherein the transfer rotation member (61) is intermittently driven such that a stop position of each of the accommodation grooves (63) of the transfer rotation member (61) at the needle piercing part (5) is more frontward in a rotational direction than the needle (51).

3. The soft sphere transfer device of Claim 1, wherein the transfer rotation member (61) is configured to be intermittently driven and rotated at a predetermined pitch in a circumferential direction, and
wherein the needle (51) of the needle piercing part (5) is disposed such that a position of the needle (51) is more rearward in a rotational direction than each of the accommodation grooves (63), which is stopped in the needle piercing part (5), of the transfer rotation member (61).

4. The soft sphere transfer device of any one of Claims 1 to 3, wherein the narrow width portion (9) includes a pair of protrusion portions protruding inward from both sides of the side walls (8A) in the width direction.

5. The soft sphere transfer device of Claim 4, wherein an inclined surface (92A) protruding inward toward a downstream side in a transfer direction of each of the protrusion portions is provided at least on an upstream side in the transfer direction.

6. A recombinant protein production method, comprising:
recovering fish eggs (e), which are soft spheres transferred by the soft sphere transfer device of any one of Claims 1 to 5;
transferring the fish eggs (e) recovered in the recovering the fish eggs (e) to a constant temperature bath and performing gene recombination for a predetermined time;
sorting the fish eggs (e) having proteins generated by performing the gene recombination; and
extracting the proteins by grinding the fish eggs (e) taken out in the sorting the fish eggs (e) and putting the fish eggs (e) into a centrifugal separator.
